# EUROPEAN PATENT APPLICATION

(11) **EP 2 857 003 A1**
(43) Date of publication of application: **08.04.2015**
(21) Application number: 13187026.3
(22) Date of filing: 02.10.2013
(51) Int. Cl.: A61K 8/41, A61K 8/46, A61Q 5/04, A61K 8/898

(54) **Hair conditioning composition comprising ammonium thioglycolate and/or ammonium thiolactate**

(71) Applicant: The Procter & Gamble Company, Cincinnati, OH 45202 (US)
(72) Inventor: Wörner, Christiane, 64274 Darmstadt (DE); Friedrich, Rainer, 64274 Darmstadt (DE)
(74) Representative: Holmes, Rosalind

(57) **Abstract**

Hair conditioning composition comprising: from about 2.0% to about 9.0% reducing agent, wherein the reducing agent is selected from the group consisting of: ammonium thioglycolate; ammonium thiolactate; and mixtures thereof; from about 0.5% to about 1.5% alkalising agent; from about 2.0% to about 10% conditioning agent selected from the group consisting of: silicone compounds; cationic surfactants; and mixtures thereof; a buffering agent. The composition has a pH of from about pH 7.5 to about pH 9.5 and a viscosity of from about 500 mPa·s to about 5500 mPa·s measured at 25°C. The composition is substantially free of an additional reducing agent capable of reducing disulfide bonds in keratin fibres. Also associated methods, processes, kits and uses.

## Description

### FIELD OF THE INVENTION

Hair conditioning composition comprising ammonium thioglycolate and/or ammonium thiolactate and a conditioning agent. Also associated methods, processes, kits and uses.

### BACKGROUND OF THE INVENTION

Products for hair straightening are known and there is demand for improved products and methods to meet consumer need for straighter hairstyles, both for home use by consumers and also by professional stylists working in the hair salon industry, which have different needs and priorities. In today's hair salon environment, the usual technology employed to straighten the hair fibres on a head of hair results in a reduction and oxidation of the disulfide bonds inside the hair fibres. The most commonly used active in this technological area is thioglycolic acid (TGA), which has been used for many years, particularly for perming (i.e. hair curling) treatments. However, there are also other reducing agents used globally which follow the same chemical principles of reduction and oxidation. More recently and mainly across North and South America, a different technological approach has become popular where the leading active used is formaldehyde. This technology is a crosslinking with the hair structure rather than a reduction/oxidation approach.

The approach using reduction and oxidation technology has two key disadvantages. Firstly, consumers colour i.e. dye their hair much more frequently than in the days when perms were popular, thus the risk of hair damage with this technology approach is higher in modem times. Secondly, today's stylists - particularly outside of Asia - do not typically receive extensive training on TGA technology anymore and if you leave either of the two key treatments of the straightening process on the hair for too long, the hair risks becoming more damaged than is acceptable. Since the hair salon, as a competitive high-street business, and the employed stylists do not want to carry any risk of damaging their reputation as a result of dissatisfied customers, the reduction and oxidation technology is typically not well-trusted.

With the crosslinking approach using formaldehyde, the key concern is safety where stylists may experience coughing and eye tearing during the very long application process. Another problem with the formaldehyde approach is that the hair needs to be exposed to very high temperatures (in the region of 450°F i.e. ∼232°C) which can be damaging for the hair fibre structure.

While consumers like to have straight hair, they are afraid of both the hair damage risk as well as the health and safety concerns the two different technologies carry. Professional hair stylists also share these concerns and risk not only the reputation of the salon, but their own professional reputation when deciding to offer such straightening treatments to their clients.

US 5,294,230 (Wu et al.*)* discloses a waving lotion comprising both ammonium thioglycolate and ammonium bisulfite. US 5,046,515 (Heinz et al.*)* discloses a permanent shaping agent for human hair and mentions an example comprising *inter alia* 12.5 g of 80% thioglycolic acid and 0.1 g cetyltrimethylammonium chloride. US 2008/0075682 (Cassier et al.) discloses a composition comprising N-alkyl-2-mercaptoacetamide and a cationic polymer for permanently shaping hair and a process therefor. US 7,078,025 (Kripp et al.*)* discloses a two-component product and method for caring for hair and permanent shaping of hair. The keratin reducing agent used in the two-component composition may be thioglycolic acid and additive ingredients mentioned include pantothenic acids, and cationic surface active agents, for example quaternary ammonium salts.

In light of this, there is a need for improved straightening products and methods for achieving straight hair. In particular, there is a need for such products and methods to be safer for the health of both the consumer that receives the treatment and stylist that applies the treatment. There is also a need for such products and methods to be gentler on the hair fibre structure such that hair damage is much less apparent and significant, particularly for previously dyed hair, and yet still provide efficacious straightening. Furthermore, there is a need for the straightening treatment to be short and easy to learn, both for the professional stylist and for the consumer at home, which have different skill sets. On the other hand, the stylist must feel empowered to adapt the treatment to the hair type of the consumer in question, the treatment selected would for example be dependent upon the hair dyeing history, damage level, hair length and needs of each consumer presented to the client. Thus, there is a need for straightening products and methods that the stylist can use their expertise and experience tailor to client need. It would also be advantageous if the straightening treatment would not require heating the hair to very high temperatures. A further need is for a straightening treatment such that the hair also has the appearance of improved health after treatment - this is particularly pertinent in the hair straightening industry where consumer and stylist confidence has been damaged by health and safety concerns.

### SUMMARY OF THE INVENTION

A first aspect relates to a hair conditioning composition comprising:
- from about 2.0% to about 9.0% reducing agent, wherein the reducing agent is selected from the group consisting of: ammonium thioglycolate; ammonium thiolactate; and mixtures thereof;
- from about 0.5% to about 1.5% alkalising agent;
- from about 2.0% to about 10% conditioning agent selected from the group consisting of: silicone compounds; cationic surfactants; and mixtures thereof;
- a buffering agent;
wherein the composition has a pH of from about pH 7.5 to about pH 9.5;
wherein the composition has a viscosity of from about 500 mPa·s to about 5500 mPa·s measured at 25°C;
wherein the composition is substantially free of an additional reducing agent capable of reducing disulfide bonds in keratin fibres.

A second aspect relates to a hair straightening method comprising:
i. analysing hair health and/or hair type;
ii. optionally applying a conditioning pre-treatment to the hair;
iii. mixing together a conditioning formulation and a straightening formulation to form a hair conditioning composition;
iv. applying the hair conditioning composition to hair;
v. allowing the hair conditioning composition to remain on the hair for a development time x;
vi. rinsing and drying the hair;
vii. mechanically straightening the hair using a temperature of from about 80°C to about 250°C;
viii. applying a neutralising formulation to the hair;
ix. rinsing the hair;
x. optionally applying a finishing formulation to the hair.

A third aspect relates to a process for obtaining a hair conditioning composition, comprising:
(I) analysing hair health and/or hair type;
(II) selecting and providing a conditioning formulation and a straightening formulation;
(III) mixing together the conditioning formulation and the straightening formulation in proportions tailored to the hair health and/or hair type.

A fourth aspect relates to a composition directly obtained by the process of the third aspect.

A fifth aspect relates to a hair straightening kit comprising:
(a) optionally a conditioning pre-treatment;
(b) a straightening formulation comprising a reducing agent;
(c) a conditioning formulation;
(d) optionally a conditioning infusion and/or a conditioning oil;
(e) optionally a neutralising formulation;
(f) optionally a finishing formulation.

A sixth aspect relates to the use of the composition of the first aspect for improved hair shine; for improved hair combing, preferably for reduced combing force; or for application onto already straightened hair.

A seventh aspect relates to a method of treating already dyed hair comprising applying a hair straightening composition to the already dyed hair, wherein the hair straightening composition comprises:
- from about 2.0% to about 9.0% reducing agent;
- from about 0.5% to about 1.5% alkalising agent;
- from about 2.0% to about 10% conditioning agent;
- a buffering agent;
wherein the composition has a pH of from about pH 7.5 to about pH 9.5;
wherein the composition has a viscosity of about 500 mPa·s to about 5500 mPa·s measured at 25°C.

### BRIEF DESCRIPTION OF THE DRAWINGS

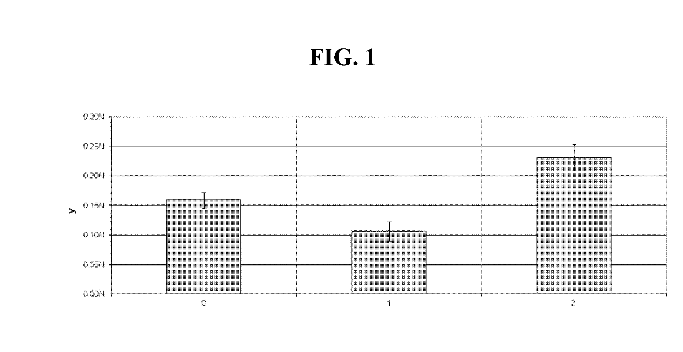

Fig 1 shows data for the force when combing wet hair for various samples. The bars are labelled from left to right: C, 1 and 2. C is the control sample; 1 is the treated sample not pursuant to the present invention; 2 is the treated sample pursuant to the present invention. The axis y is combing force in Newtons.

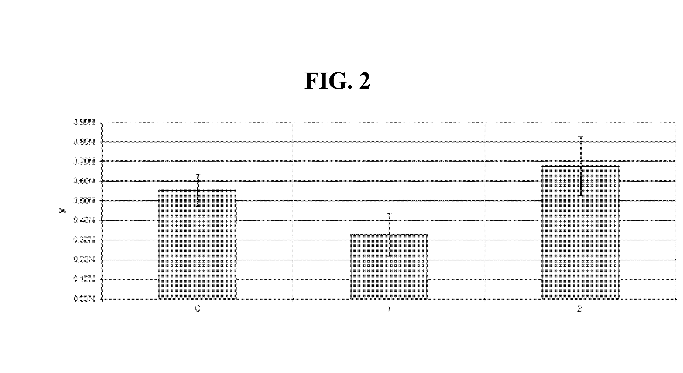

Fig 2 shows data for the force when combing dry hair for various samples. The bars are labelled from left to right: C, 1 and 2. C is the control sample; 1 is the treated sample not pursuant to the present invention; 2 is the treated sample pursuant to the present invention. The axis y is combing force in Newtons.

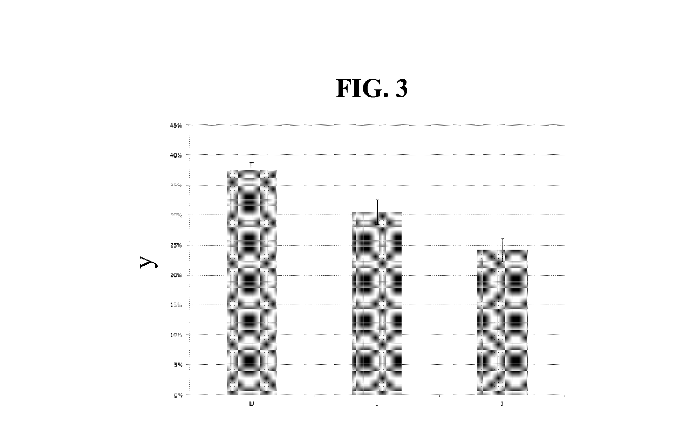

Fig 3 shows data for the shine percentage on various samples. The bars are labelled from left to right: U, 1 and 2. U is the control sample; 1 is the treated sample not pursuant to the present invention; 2 is the treated sample pursuant to the present invention. The axis y is the shine in %.

### DETAILED DESCRIPTION OF THE INVENTION

### Definitions

In this document, including in all embodiments of all aspects of the present invention, the following definitions apply unless specifically stated otherwise. All percentages are by weight of the total composition or total formulation. All ratios are weight ratios. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams". All weights as they pertain to listed ingredients are based on the active level and do not include carriers or byproducts that may be included in commercially available materials. Herein, "comprising" means that other steps and other ingredients can be in addition. "Comprising" encompasses the terms "consisting of' and "consisting essentially of'. The compositions, formulations, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

"Molecular weight" or "M.Wt." or "MW" and grammatical equivalents mean the number average molecular weight.

"Water-soluble" refers to any material that is sufficiently soluble in water to form a clear solution to the naked eye at a concentration of 0.1% by weight of the material in water at 25°C. The term "water-insoluble" refers to any material that is not "water-soluble".

"Dry" or "substantially dry" means comprising less than 5%, less than 3% or, less than 2%, less than 1 %, or about 0% of any compound or composition being in liquid form when measured at 25°C at ambient conditions. Such compounds or compositions being in liquid form include water, oils, organic solvents and other wetting agents. "Anhydrous" means that the composition comprises less than 5%, less than 3% or, less than 2%, less than 1%, or about 0% water by total weight of the composition.

"Substantially free from" or "substantially free of" means less than about 1%, or less than 0.8%, or less than 0.5%, or less than 0.3%, or about 0%, by total weight of the composition or formulation.

"Hair" means mammalian keratin fibres including scalp hair, facial hair and body hair. It includes such hair still being attached to a living subject and also hair that has been removed there from such as hair swatches and hair on a doll/mannequin. In an embodiment, "hair" means human hair. "Hair shaft" or "hair fibre" means an individual hair strand and may be used interchangeably with the term "hair."

"Proximal to the scalp" means that portion of an extended, or substantially straightened, hair shaft that is closer in distance to the scalp than to the end of the hair. Thus, about 50% of the hair fibre length would be considered proximal to the scalp, and about 50% of the hair fibre would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

"Chemically modify" or grammatical equivalents thereof, means that a chemical moiety such as monomer and/or crosslinker and/or polymer, stably affixes to a second chemical moiety, for example, a keratin protein, another component of hair, and/or another monomer or crosslinker or polymer. Normally, "chemically modify" means stably affix via a covalent bond, unless otherwise stated.

"Cosmetically acceptable" means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives" includes but is not limited to, amide, ether, ester, amino, carboxyl, acetyl, acid, salt and/or alcohol derivatives of a given compound. In an embodiment, "derivatives thereof' means the amide, ether, ester, amino, carboxyl, acetyl, acid, salt and alcohol derivatives.

"Monomer" means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit" means a monomer that has already been polymerised i.e. is part of a polymer.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers.

"Hairstyling polymer" means a hair-fixing polymer which forms a film on a surface i.e. a film-forming polymer. 'Hairstyling polymer' and 'film-forming polymer' are used interchangeably in the art. In the context of hair science, this surface is the surface of individual hair fibres or a plurality thereof. The hairstyling polymer causes the hair fibres to be glued together to build welds, which are effectively crosslinks that provide the hold benefit. In concert, these welds form a 'hairnet' to provide hair hold and volume benefits to the consumer. When the net of welds is effectively formed, the hold and volume benefits can last all day and offer good resistance to environmental humidity.

"Kit" means a package comprising a plurality of components. "Kit" may be referred to as "kit-of-parts". An example of a kit is, for example, a first composition and a separately packaged second composition and optionally application instructions.

"Hair straightening" includes hair relaxing, hair smoothing, hair de-curling and the like.

### Explanation of the Invention

The inventors have carefully selected a specific combination of actives that results in safe straightening and intensive conditioning combined in one treatment. The hair straightening result provided is milder than the straightening result provided by other products known on the market, but still provides efficacious and noticeable straightening, and importantly without concerns of major hair structure damage. The intensive conditioning actives provide a soft hair feel when touched and excellent hair shine. The pH range selected means improved penetration of the conditioning actives into the hair fibre, and thus a more intensive conditioning effect than just using a conventional intensive mask as a post-treatment step. Indeed, by applying the straightening and the conditioning actives at high pH levels, the conditioning actives are able to penetrate deep inside the hair fibre and become locked inside the hair following an oxidation step which has a low pH and closes the hair structure again. Thus, elements of both durable straightening and durable conditioning is provided by the present invention.

In addition, a relatively low level of ammonia is employed, which makes the treatment experience more pleasant for both consumer and stylist. Furthermore, only thioglycolate actives are utilised as reducing agents in the composition of the present invention, which correlates with the mildness of the treatment reduces the risk and possibilities for unwanted side reactions. Moreover, the viscosity of the composition of present invention means that there is a reduced likelihood for the composition to drip.

The risk of hair damage associated with the straightening treatment can be easily explained to and also accepted by the consumer since the risk level is typically the same as that of a standard semi-permanent dyeing treatment. Semi-permanent dye products are normally considered to be low-risk products by consumers that are interested in hair straightening products and treatments.

The present invention is very attractive and suitable for use by professional stylists in a salon because it combines the best of both worlds: effective and safe hair straightening and simultaneous intensive conditioning. Such a cross-category product is rare in the hair treatment market and thus provides a unique selling point. The hair salon industry is a multi-million dollar industry and salons must compete with both each other and also with retail products to win over consumers. Thus, products and methods sold by salons must offer something special. Another significant advantage of the present invention is the customisability of the treatment. The stylist can analyse the hair type and treatment history of their client and use their own skills and experience, relationship with their client, as well as their personal creativity, to choose the optimal and tailored combination of actives to meet consumer demand. This is an especially attractive opportunity for the professional stylist.

The uniqueness of the present invention can be explained by the fact that it is conventionally known that certain conditioning actives are not stable at high pHs and also that certain conditioning actives can react with certain straightening actives, particularly in combination with certain higher temperature ranges. This can lead to non-favourable side effects and side reactions, the result of which is poor efficacy and unacceptable results. Thus it is not possible to simply combine any known straightening product and any known conditioning product and expect acceptable results. This is a known bias of the formulation technologist. Furthermore, there is a link between intensive conditioning treatments and low pH. Indeed, most conditioner products contain acids and exhibit an acidic pH. For example, following hair dyeing, a low pH intensive conditioning mask is usually employed following application of the typically alkaline hair dye as a means of neutralisation. In addition, there is a known link between low-pH conditioners and shine benefits. Moreover, due to health and safety concerns that have been given high media attention in the hair straightening industry, the formulation technologist is sensitive to the need for highly safe and low-risk products and methods. In addition, consumers may have treated their hair with number of dye products, thus the base chemistry of the hair fibre of each consumer, is not known to the formulation technologist. Thus a cross-category product is not an obvious choice for the formulation technologist.

In summary, the present invention provides effective, safer and lower risk hair straightening and simultaneous intensive conditioning, where the treatment is easy and fast to execute, customisable, and easily understood by both consumer and stylist.

The different aspects of the invention and how they are interrelated are summarised as follows. The first aspect relates to a hair conditioning composition. The second aspect relates to a hair straightening method. The second aspect comprises mixing together a conditioning formulation and a straightening formulation to form a hair conditioning composition. The hair conditioning composition mentioned in the second aspect may be the same as that described in the first aspect. The third aspect relates to a process for obtaining a hair conditioning composition. The hair conditioning composition mentioned in the third aspect may be the same as that described in the first aspect. The fourth aspect relates to the composition directly obtained by the process of the third aspect. The composition may be the hair conditioning composition of the first aspect. The sixth aspect relates to a hair straightening kit. The sixth aspect relates to various specific uses of the composition according to the first aspect or of the fourth aspect. The seventh aspect relates to a method of treating already dyed hair. The seventh aspect may employ the hair conditioning composition of the first aspect or the composition of the fourth aspect.

The details of the present invention are hereinafter described.

### Reducing Agent

In an embodiment, the composition/formulation comprises reducing agent. In an embodiment, the hair conditioning composition comprises from about 2.0% to about 9.0% reducing agent, In an embodiment, the reducing agent is selected from the group consisting of ammonium thioglycolate, ammonium thiolactate, and mixtures thereof. The selected reducing agents have been chosen in view of the fact that they are well-known in hair treating formulations such as perming formulations. This has the effect that these compounds are well-characterised from a toxicology standpoint, easily available and well-understood by stylists.

The amount of reducing agent is critical for ensuring sufficient straightening effect without risking hair damage. In an embodiment, the hair conditioning composition comprises from about 2.25%, or from about 2.5%, or from about 2.75%, or from about 3.0%, or from about 3.25%, or from about 3.5%, or from about 3.75%, or from about 4.0%, or from about 4.25%, or from about 4.5%, or from about 4.75%, or from about 5.0% to about 8.75%, or to about 8.5% or to about 8.25% or to about 8.0% or to about 7.75%, or to about 7.5%, or to about 7.25%, or to about 7.0%, or to about 6.75%, or to about 6.5%, or to about 6.25%, or to about 6.0%, or to about 5.75%, or to about 5.5%, or to about 5.25%, or to about 5.0% reducing agent. This amount may be the total amount of reducing agent present in the composition.

In an embodiment, the composition/formulation further comprises a dithioglycolate compound and/or a dithiolactate compound. In an embodiment, the composition/formulation further comprises diammonium dithioglycolate and/or diammonium dithiolactate. The dimeric versions of ammonium thiolactate and ammonium thiolactate, namely diammonium dithioglycolate and diammonium dithiolactate, respectively, are useful in that they help regulate the availability of the thioglycolate ion or thiolactate ion to the hair via the formation of an equilibrium. The thioglycolate ion has the chemical formula HS-CH₂-COO⁻ and the thiolactate ion has the chemical formula HS-CH(CH₃)-COO⁻.

### Conditioning agent

In an embodiment, the composition/formulation comprises conditioning agent. In an embodiment, the hair conditioning composition comprises a conditioning agent selected from the group consisting of: silicone polymer; a cationic surfactant; and mixtures thereof. In an embodiment, hair conditioning formulation comprises from about 2.0% to about 10% conditioning agent. The amount of conditioning agent in the hair conditioning composition is important in view of the final effect on hair being having certain characteristics - for example over 10% conditioning agent in the hair conditioning composition would likely result in the hair being weighed down and too heavy. On the other hand, less than 2% conditioning agent would likely not have enough conditioning effects on hair i.e. hair has too much friction, tangles easily, is too 'flyaway', lacks shine etc. In an embodiment, hair conditioning composition comprises from about 2.5%, or from 3.0%, or from 3.5%, or from 4.0% to about 9.5%, or to about 9.0%, or to about 8.5%, or to about 8.0%, or to about 7.5%, or to about 7.0%, or to about 6.5%, or to about 6.0%, or to about 5.5%, or to about 5.0%, or to about 4.5%, or to about 4.0%, or to about 3.5% conditioning agent. In an embodiment, the hair conditioning formulation comprises as conditioning agent a mixture of silicone polymer and cationic surfactant.

In an embodiment, conditioning formulation comprises from about 2.5%, or from 3.0%, or from 3.5%, or from 4.0%, or from about 4.5%, or from about 5.0%, or from about 5.5%, or from about 6.0%, or from about 6.5% to about 12.5%, or to about 12.0%, or to about 11.5%, or to about 11.0%, or to about 10.5%, or to about 10.0%, or to about 9.5%, or to about 9.0%, or to about 8.5%, or to about 8.0%, or to about 7.5% conditioning agent. In an embodiment, the conditioning formulation comprises as conditioning agent a mixture of silicone polymer and cationic surfactant.

In an embodiment, the conditioning formulation comprises cationic surfactant. In an embodiment, the hair conditioning composition comprises cationic surfactant. The composition/formulation may comprise from about 0.05% to about 3.5%, or from about 0.1% to about 3.0%, or from about 0.5% to about 2.5%, or from about 1.0% to about 2.0% cationic surfactant. In an embodiment, cationic surfactant is according to Formula II: 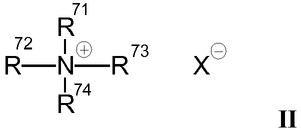 wherein at least one of R⁷¹, R⁷², R⁷³ and R⁷⁴ is selected from: an aliphatic group of from 8 to 30 carbon atoms; an aromatic, alkoxy, polyoxyalkylene, alkylamido, hydroxyalkyl, aryl; or an alkylaryl group having from 7 to 22 carbon atoms; wherein the remainder of R⁷¹, R⁷², R⁷³ and R⁷⁴ are independently selected from the group consisting of: an aliphatic group consisting of from 1 to 22 carbon atoms; and an aromatic, alkoxy, polyoxyalkylene, alkylamido, hydroxyalkyl, aryl or alkylaryl group having up to 22 carbon atoms; wherein X is selected from the group consisting of: halogen, acetate, citrate, lactate, glycolate, phosphate, nitrate, sulfonate, sulfate, alkylsulfate, alkyl sulfonate radicals, and mixtures thereof.

In an embodiment, cationic surfactant is according to Formula II (see above), wherein at least one of R⁷¹, R⁷², R⁷³ and R⁷⁴ is an aliphatic group having from 16 to 24 carbon atoms; wherein the remainder of R⁷¹, R⁷², R⁷³ and R⁷⁴ are independently selected from the group consisting of aliphatic groups having from 1 to 4 carbon atoms; wherein X is selected from the group consisting of: chloride or sulfate.

In an embodiment, the cationic surfactant is selected from the group consisting of: behenyltrimethylammonium chloride, methyl sulfate or ethyl sulfate; stearyltrimethylammonium chloride, methyl sulfate or ethyl sulfate; and mixtures thereof. It is believed that a longer alkyl group provides improved smoothness and soft feeling on wet and dry hair, compared to cationic surfactants with a shorter alkyl group. It is also believed that such cationic surfactants can provide reduced scalp irritation, compared to those having a shorter alkyl group.

The cationic surfactant may be a di-long alkyl quaternized ammonium salt selected from the group consisting of: dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In an embodiment, the cationic surfactant is a tertiary amidoamine having an alkyl group of from about 12 to about 22 carbons. The tertiary amidoamine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethyl-, behenamidopropyldiethyl-, behenamidoethyldiethyl-, behenamidoethyldimethyl-, arachidamidopropyldimethyl-, arachidamidopropyldiethyl-, arachidamidoethyldiethyl-, and arachidamidoethyldimethyl-amine, diethylaminoethylstearamide, and mixtures thereof.

A tertiary amidoamine may be used in combination with an acid. The acid is typically used as a salt-forming anion. In an embodiment, the acid is selected from the group consisting of: lactic acid, malic acid, hydrochloric acid, 1-glumatic acid, acetic acid, citric acid, and mixtures thereof.

In an embodiment, the cationic surfactant is selected from the group consisting of: cetyltrimonium chloride (CTAC), stearyltrimonium chloride (STAC), behentrimonium methosulfate, behentrimonium chloride, stearoylamidopropyldimethyl amine (SAPDMA), distearyldimethylammonium chloride, and mixtures thereof. In an embodiment, the hair conditioning composition comprises a mixture of cetyltrimonium chloride (CTAC) and behentrimonium chloride.

In an embodiment, the composition/formulation comprises silicone compound. In an embodiment, the silicone compound is a silicone polymer. In an embodiment, the silicone polymer is a terminal aminosilicone. "Terminal aminosilicone" as defined herein means a silicone polymer comprising one or more amino groups at one or both ends of the silicone backbone. In an embodiment, the conditioning formulation comprises terminal aminosilicone. In an embodiment, the hair conditioning composition comprises terminal aminosilicone.

In an embodiment, the composition/formulation is substantially free of any silicone compound comprising pendant amino groups. In an embodiment, the composition/formulation is substantially free of any silicone compound other than terminal aminosilicones. In an embodiment, the amino group at least one terminus of the silicone backbone of the terminal aminosilicone is selected from the group consisting of: primary amines, secondary amines and tertiary amines. The terminal aminosilicone may conform to Formula III:

(R₁)ₐG₃₋ₐ-Si-(-OSiG₂)ₙ-O-SiG₃₋ₐ(R₁)ₐ III

wherein G is hydrogen, phenyl, hydroxy, or C₁-C₈ alkyl, preferably methyl; a is an integer having a value from 1 to 3, or is 1; b is 0, 1 or 2, or is 1; n is a number from 0 to 1,999; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A ; -N(R₂)CH₂-CH₂-NR₂H₂A; wherein R₂ is hydrogen, phenyl, benzyl, or a saturated hydrocarbon radical; A is a halide ion. In an embodiment, R₂ is an alkyl radical having from 1 to 20 carbon atoms, or from 2 to 18 carbon atoms, or from 4 to 12 carbon atoms.

A suitable terminal aminosilicone corresponding to Formula III has a=1, q=3, G=methyl, n is from about 1000 to about 2500, alternatively from about 1500 to about 1700; and L is-N(CH₃)₂. A suitable terminal aminosilicone corresponding to Formula III has a=0, G=methyl, n is from about 100 to about 1500, or from about 200 to about, L is selected from the following groups: -N(R₂)CH₂-CH₂-N(R₂)₂; -N(R₂)₂; -N(R₂)₃A ; -N(R₂)CH₂-CH₂-NR₂H₂A ; wherein R₂ is hydrogen, phenyl, benzyl, or a saturated hydrocarbon radical; A is a halide ion, alternatively L is -NH₂. In an embodiment, R₂ is an alkyl radical having from 1 to 20 carbon atoms, or from 2 to 18 carbon atoms, or from 4 to 12 carbon atoms. In an embodiment, the terminal aminosilicone is selected from the group consisting of bis-aminomethyl dimethicone, bis-aminoethyl dimethicone, bis-aminopropyl dimethicone, bis-aminobutyl dimethicone, and mixtures thereof. In an embodiment, the viscosity of the terminal aminosilicone is from about 1,000 to about 30,000 cPs, or from about 5,000 to about 20,000 cPs, or from about 8,000 cPs to about 12,000 cPs measured at 25°C.

In an embodiment, the silicone polymer is selected from the group consisting of: polydimethylsiloxane; polydiethylsiloxane; polymethylphenylsiloxane; and mixtures thereof. In an embodiment, the silicone polymer is a polydimethylsiloxane. These silicone compounds are available, for example, from the General Electric Company in their ViscasilR and SF 96 series, and from Dow Coming in their Dow Coming 200 series.

In an embodiment, the silicone polymer has a viscosity of from about 1,000 to about 2,000,000 centistokes, or from about 10,000 to about 1,800,000 centistokes, or from about 25,000 to about 1,500,000 centistokes, measured at 25°C. The viscosity can be measured by means of a glass capillary viscometer as set forth in Dow Coming Corporate Test Method CTM0004, July 20, 1970, which is incorporated by reference herein in its entirety.

The composition/formulation may comprise from about 0.1 % to about 10%, or from about 0.5% to about 8%, or from about 1% to about 6%, or from about 1.5% to about 5.5%, or from about 2.0% to about 5.0%, or from about 2.5% to about 4.5%, or from about 3.0% to about 4.0% silicone polymer. The composition/formulation may comprise from about 0.1 % to about 10%, or from about 0.5% to about 8%, or from about 1% to about 6%, or from about 1.5% to about 5.5%, or from about 2.0% to about 5.0%, or from about 2.5% to about 4.5%, or from about 3.0% to about 4.0% terminal aminosilicone. In an embodiment, the conditioning formulation comprises from about 3.0% to about 10.0%, or from about 3.5% to about 7.0%, or from about 4.0% to about 6.5%, or from about 4.5% to about 6.0% terminal aminosilicone. In an embodiment, the hair conditioning composition comprises from about 1.0% to about 5.0%, or from about 1.5% to about 4.5%, or from about 2.0% to about 4.0%, or from about 2.5% to about 3.5%, or from about 3.0% to about 3.5% terminal aminosilicone.

In an embodiment, the hair conditioning composition comprises a mixture of polydimethylsiloxane and terminal aminosilicone.

### Fatty compound

In an embodiment, the composition/formulation comprises fatty compound. The fatty compound provides additional conditioning benefits on top of those provided by the conditioning agent as described herein. In embodiment, the composition/formulation comprises a cationic surfactant, a terminal aminosilicone and a high melting point fatty compound. In an embodiment, the cationic surfactant and the high melting point fatty compound are contained at a level such that the weight ratio of the cationic surfactant to the high melting point fatty compound is in the range of from about 1:1 to about 1:10, or from about 1:1 to about 1:6.

The high melting point fatty compound useful herein has a melting point of 25°C or higher, and is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. In an embodiment, the composition/formulation comprises from about 0.1 % to about 16%, or from about 1% to about 14%, or from about 1.5% to about 10%, or from about 2% to about 8% of high melting point fatty compound. This amount is in view of providing improved conditioning benefits such as slippery feel during the application to wet hair, softness and moisturized feel on dry hair.

In an embodiment, the high melting point fatty compound is fatty alcohol. In an embodiment, the fatty alcohol is selected from the group consisting of: cetyl alcohol, stearyl alcohol, behenyl alcohol, and mixtures thereof. In an embodiment, the fatty alcohol is a mixture of cetyl alcohol and stearyl alcohol. In an embodiment, the weight ratio of total fatty alcohol to terminal aminosilicone is from about 0.5:1 to about 10:1, or from about 1:1 to about 5:1, or from about 2.4:1 to about 2.7:1.

### Carrier

The composition/formulation may be in the form of a pourable liquid (pourable when under ambient conditions). In an embodiment, the composition/formulation comprises a cosmetically acceptable aqueous carrier and is in the form of a pourable liquid. The composition/formulation may comprise a cosmetically acceptable aqueous carrier present at a level of from about 20% to about 95%, or from about 60% to about 85%. The cosmetically acceptable aqueous carrier may be selected from the group consisting of water and water solutions of lower alkyl alcohols and polyhydric alcohols. The lower alkyl alcohols may be monohydric alcohols having 1 to 6 carbons. In an embodiment, the lower alkyl alcohols are ethanol and isopropanol. The polyhydric alcohols may be propylene glycol, hexylene glycol, glycerin, and propane diol.

### Optional compounds

The composition/formulation may further comprise: water soluble vitamins and their derivatives, water-soluble amino acids and their salts and/or derivatives, viscosity modifiers, dyes, nonvolatile solvents or diluents (water-soluble and water-insoluble), pearlescent aids, thickeners, foam boosters, additional surfactants or nonionic co-surfactants, pediculocides, pH adjusting agents, perfumes, preservatives, chelants, proteins, skin active agents, sunscreens, UV absorbers, vitamins, niacinamide, caffeine and minoxidil. The composition/formulation may comprise from about 0%, or from about 0.1% to about 5% vitamins and amino acids. The composition/formulation may also comprise pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C.I. Names. The composition/formulation may comprise from about 0%, or from about 0.1% to about 5% pigment materials. The composition/formulation may comprise from about 0%, or from about 0.1 % to about 5% antimicrobial agents. In an embodiment, the composition/formulation comprises an organic acid selected from the group consisting of: glycine, L-methionine, L-arginine, biotin, creatine, and mixtures thereof. In an embodiment, the composition/formulation comprises an antidandruff agent. In an embodiment, the composition/formulation comprises zinc pyrithione. In an embodiment, the composition/formulation comprises panthenol. In an embodiment, the composition/formulation comprises a wax compound. In an embodiment, the composition/formulation comprises beeswax.

### Thickening polymer

In an embodiment, the composition/formulation comprises thickening polymer. In an embodiment, the thickening polymer is a water-soluble cationic polymer. In an embodiment, the thickening polymer is a polyquaternium compound. In an embodiment, the polyquaternium compound is selected from the group consisting of: polyquaternium-2, polyquaternium-4, polyquaternium-5, polyquaternium-6, polyquaternium-7, polyquaternium-8, polyquaternium-10, polyquaternium-11, polyquaternium-16, polyquaternium-17, polyquaternium-18, polyquaternium-28, polyquaternium-32, polyquaternium-37, polyquaternium-42, polyquaternium-43, polyquaternium-44, polyquaternium-46, polyquaternium-55, polyquaternium-59, and mixtures thereof. In an embodiment, the polyquaternium compound is selected from the group consisting of: polyquaternium-6, polyquaternium-7, polyquaternium-11, polyquaternium-16, polyquaternium-44, polyquaternium-46, polyquaternium-55, and mixtures thereof.

In an embodiment, the composition/formulation comprises from about 0.001 % to about 3%, or from about 0.01% to about 2%, or from about 0.1% to about 1.5%, or from about 0.2% to about 1.25%, or from about 0.3% to about 1.0%, or from about 0.4% to about 0.8% thickening polymer.

### Surfactant

In an embodiment, the composition/formulation comprises a surfactant. In an embodiment, the composition/formulation comprises a non-ionic surfactant. In an embodiment, the non-ionic surfactant is a ceteareth compound. In an embodiment, the ceteareth compound having from 2 to 100, or from 5 to 45, or from 10 to 30 ethylene oxide residues in the polyoxyethylene chain. In an embodiment, the composition/formulation comprises from about 0.1 % to about 10%, or from about 1% to about 5% surfactant.

### Viscosity

In an embodiment, the composition/formulation has a viscosity of from about 500 mPa·s to about 5500 mPa·s measured at 25°C. In an embodiment, the viscosity is from about 700 mPa·s, or from about 900 mPa·s, or from about 1100 mPa·s, or from about 1300 mPa·s, or from about 1500 mPa·s, or from about 1700 mPa·s, or from about 1900 mPa·s, or from about 2100 mPa·s, or from about 2200 mPa·s, or from about 2400 mPa·s, or from about 2500 mPa·s, or from about 2700 mPa·s, or from about 2900 mPa·s, or from about 3100 mPa·s, to about 5300 mPa·s, or to about 5100 mPa·s, or to about 4900 mPa·s, or to about 4700 mPa·s, or to about 4500 mPa·s, or to about 4300 mPa·s, or to about 4100 mPa·s, or to about 3900 mPa·s, or to about 3700 mPa·s, or to about 3500 mPa·s, or to about 3300 mPa·s, or to about 3100 mPa·s, or to about 2900 mPa·s, or to about 2500 mPa·s, or to about 2300 mPa·s measured at 25°C. In an embodiment, the viscosity is from about 3000 mPa·s to about 4500 mPa·s measured at 25°C.

The viscosity in mPa·s is measured with a Haake Rheometer VT-550, Measure system: MV-DIN, Shear rate: 12.9 s⁻¹. The viscosity is measured at 25°C.

The viscosity of the composition/formulation is important in view of the avoidance of dripping during use and application onto hair. This is particularly in the case of the hair conditioning composition, which provides the on-hair conditioning combined with straightening effects. Indeed, in a hair straightening method as described herein, a conditioning formulation and the straightening formulation are mixed. It is advantageous of the conditioning formulation and the straightening formulation have similar viscosities in view of efficient and fast mixing. In an embodiment, both the conditioning formulation and the straightening formulation have a viscosity of from about 500 mPa·s to about 5500 mPa·s measured at 25°C. In an embodiment, both the conditioning formulation and the straightening formulation have a viscosity of from about 700 mPa·s, or from about 900 mPa·s, or from about 1100 mPa·s, or from about 1300 mPa·s, or from about 1500 mPa·s, or from about 1700 mPa·s, or from about 1900 mPa·s, or from about 2100 mPa·s, or from about 2200 mPa·s, or from about 2400 mPa·s, or from about 2500 mPa·s, or from about 2700 mPa·s, or from about 2900 mPa·s, or from about 3100 mPa·s, to about 5300 mPa·s, or to about 5100 mPa·s, or to about 4900 mPa·s, or to about 4700 mPa·s, or to about 4500 mPa·s, or to about 4300 mPa·s, or to about 4100 mPa·s, or to about 3900 mPa·s, or to about 3700 mPa·s, or to about 3500 mPa·s, or to about 3300 mPa·s, or to about 3100 mPa·s, or to about 2900 mPa·s, or to about 2500 mPa·s, or to about 2300 mPa·s measured at 25°C. In an embodiment, the both the conditioning formulation and the straightening formulation have a viscosity of from about 3000 mPa·s to about 4500 mPa·s measured at 25°C.

### pH, alkalising agent, buffer

In an embodiment, the composition/formulation comprises alkalising agent. In an embodiment, the hair conditioning composition comprises alkalising agent. The alkalising agent is useful in keeping the pH high enough, for example above pH 7. In an embodiment the alkalising agent is ammonia. In an embodiment, the composition/formulation comprises from about 0.1% to about 3%, or from about 0.2% to about 1.9%, or from about 0.3% to about 1.8%, or from about 0.4% to about 1.7%, or from about 0.4% to about 1.7%, or from about 0.5% to about 1.6%, or from about 0.6% to about 1.5%, or from about 0.7% to about 1.4%, or from about 0.8% to about 1.3%, or from about 0.9% to about 1.2% ammonia. Ammonia is useful as alkalising agent because it is well-known to the stylist and cosmetic formulator.

In an embodiment, the composition/formulation comprises buffering agent. The buffering agent is useful in keeping the pH within the specified or preferred range. In an embodiment, the buffering agent comprises ammonium ions. In an embodiment, the buffering agent comprises carbonate ions. In an embodiment, the buffering agent is ammonium bicarbonate.

In an embodiment, the composition/formulation has a pH of from about pH 7.5 to about pH 9.5. In an embodiment, hair conditioning formulation has a pH of from about pH 7.5 to about pH 9.5, or from about pH 7.75 to about pH 9.25, or from about pH 8.0 to about pH 9.0.

### Unpreferred compounds

If present, a number of compounds would result in reduced performance or other unwanted side effects if present in the composition. In an embodiment, all compositions and formulations herein are substantially free of formaldehyde. In an embodiment, the composition/formulation is substantially free of monoethanolamine.

### Hair conditioning composition

The first aspect and other aspects relate to a hair conditioning composition. In an embodiment, the hair conditioning composition comprises reducing agent, alkalising agent, conditioning agent, and buffering agent. Such agents are described herein. In an embodiment, hair conditioning composition comprises: from about 2.0% to about 9.0% reducing agent, wherein the reducing agent is selected from the group consisting of: ammonium thioglycolate; ammonium thiolactate; and mixtures thereof; from about 0.5% to about 1.5% alkalising agent; from about 2.0% to about 10% conditioning agent selected from the group consisting of: silicone compounds; cationic surfactants; and mixtures thereof; and a buffering agent. In an embodiment, the hair conditioning composition is substantially free of an additional reducing agent capable of reducing disulfide bonds in keratin fibres. In an embodiment, the composition comprises a mixture of polydimethylsiloxane and terminal aminosilicone. In an embodiment, said additional reducing agent is selected from the group consisting of: cysteine, monoethanolamine thioglycolate, thiosulfates, sulfites, bisulfites, carbonyl group-containing compounds and mixtures thereof. The term "carbonyl group" as used herein means an aldehyde (-C(=O)-H) or a ketone (-CHₓ-C(=O)-CHₓ-). The presence of an additional reducing again is not preferred in view of preserving the mildness of the hair conditioning composition. In an embodiment, wherein the composition is substantially free of monoethanolamine. the composition comprises a thickening polymer, and wherein the thickening polymer is a polyquaternium compound.

In an embodiment, the hair conditioning composition has a viscosity is from about 700 mPa·s, or from about 900 mPa·s, or from about 1100 mPa·s, or from about 1300 mPa·s, or from about 1500 mPa·s, or from about 1700 mPa·s, or from about 1900 mPa·s, or from about 2100 mPa·s, or from about 2200 mPa·s, or from about 2400 mPa·s, or from about 2500 mPa·s, or from about 2700 mPa·s, or from about 2900 mPa·s, or from about 3100 mPa·s, to about 5300 mPa·s, or to about 5100 mPa·s, or to about 4900 mPa·s, or to about 4700 mPa·s, or to about 4500 mPa·s, or to about 4300 mPa·s, or to about 4100 mPa·s, or to about 3900 mPa·s, or to about 3700 mPa·s, or to about 3500 mPa·s, or to about 3300 mPa·s, or to about 3100 mPa·s, or to about 2900 mPa·s, or to about 2500 mPa·s, or to about 2300 mPa·s measured at 25°C. In an embodiment, the viscosity is from about 3000 mPa·s to about 4500 mPa·s measured at 25°C.

In an embodiment, hair conditioning formulation has a pH of from about pH 7.5 to about pH 9.5, or from about pH 7.75 to about pH 9.25, or from about pH 8.0 to about pH 9.0.

In an embodiment, the hair conditioning composition is for depositing a terminal aminosilicone onto hair, or for providing improved deposition of the terminal aminosilicone onto hair.

In an embodiment, the hair conditioning composition comprises a cationic surfactant. In an embodiment, the cationic surfactant is according to Formula II 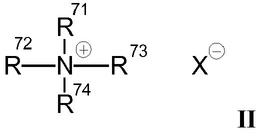 wherein at least one of R⁷¹, R⁷², R⁷³ and R⁷⁴ is an aliphatic group having from 16 to 24 carbon atoms; wherein the remainder of R⁷¹, R⁷² , R⁷³ and R⁷⁴ are independently selected from the group consisting of aliphatic groups having from 1 to 4 carbon atoms; wherein X is selected from the group consisting of: chloride or sulfate.

### Straightening formulation

Aspects of the present invention employ or relate to a straightening formulation. In an embodiment, the straightening formulation comprises a reducing agent. In an embodiment, the reducing agent is selected from the group consisting of: ammonium thioglycolate; ammonium thiolactate; and mixtures thereof. In an embodiment, the straightening formulation comprises from about 4.0% to about 20% reducing agent. In an embodiment, the reducing agent is ammonium thioglycolate and the formulation further comprises a dithioglycolate compound. In an embodiment, the straightening formulation comprises from about 4.5%, or from about 5.0%, or from about 5.5%, or from about 6.0%, or from about 6.5%, or from about 7.0%, or from about 7.5%, or from about 8.0%, or from about 8.5%, or from about 9.0%, or from about 9.5%, or from about 10.0% to about 19.5%, or to about 19%, or to about 18.5%, or to about 18.0%, or to about 17.5%, or to about 17.0%, or to about 16.5%, or to about 16.0%, or to about 15.5%, or to about 15%, or to about 14.5%, or to about 14.0%, or to about 13.5%, or to about 13.0%, or to about 12.5%, or to about 12.0%, or to about 11.5%, or to about 11.0%, or to about 10.5%, or to about 10.0%. The amount of reducing agent is critical for ensuring sufficient straightening effect without risking hair damage.

In an embodiment, the straightening composition has a viscosity of from about 500 mPa·s to about 5500 mPa·s measured at 25°C. In an embodiment, the staightening formulation has a viscosity is from about 700 mPa·s, or from about 900 mPa·s, or from about 1100 mPa·s, or from about 1300 mPa·s, or from about 1500 mPa·s, or from about 1700 mPa·s, or from about 1900 mPa·s, or from about 2100 mPa·s, or from about 2200 mPa·s, or from about 2400 mPa·s, or from about 2500 mPa·s, or from about 2700 mPa·s, or from about 2900 mPa·s, or from about 3100 mPa·s, to about 5300 mPa·s, or to about 5100 mPa·s, or to about 4900 mPa·s, or to about 4700 mPa·s, or to about 4500 mPa·s, or to about 4300 mPa·s, or to about 4100 mPa·s, or to about 3900 mPa·s, or to about 3700 mPa·s, or to about 3500 mPa·s, or to about 3300 mPa·s, or to about 3100 mPa·s, or to about 2900 mPa·s, or to about 2500 mPa·s, or to about 2300 mPa·s measured at 25°C. In an embodiment, the viscosity is from about 3000 mPa·s to about 4500 mPa·s measured at 25°C. In an embodiment, the straightening formulation is substantially free of an additional reducing agent capable of reducing disulfide bonds in keratin fibres. In an embodiment, said additional reducing agent is selected from the group consisting of: cysteine, monoethanolamine thioglycolate, thiosulfates, sulfites, bisulfites, carbonyl group-containing compounds and mixtures thereof.

### Conditioning formulation

Aspects of the present invention employ or relate to a conditioning formulation. In an embodiment, the conditioning formulation comprises a conditioning agent. In an embodiment, the conditioning agent selected from the group consisting of: silicone compounds; cationic surfactants; and mixtures thereof. Such conditioning agents are described herein. In an embodiment, the conditioning formulation comprises a silicone polymer and an alphatic hydrocarbon having from 8 to 18, or from 10 to 14 carbon atoms. In an embodiment, the conditioning formulation comprises a mixture of silicone polymers. In an embodiment, the conditioning formulation comprises a mixture of silicone polymers, a fatty alcohol, panthenol and a cationic surfactant. In an embodiment, both the conditioning formulation has a viscosity of from about 500 mPa·s to about 5500 mPa·s measured at 25°C. In an embodiment, the conditioning formulation has a viscosity is from about 700 mPa·s, or from about 900 mPa·s, or from about 1100 mPa·s, or from about 1300 mPa·s, or from about 1500 mPa·s, or from about 1700 mPa·s, or from about 1900 mPa·s, or from about 2100 mPa·s, or from about 2200 mPa·s, or from about 2400 mPa·s, or from about 2500 mPa·s, or from about 2700 mPa·s, or from about 2900 mPa·s, or from about 3100 mPa·s, to about 5300 mPa·s, or to about 5100 mPa·s, or to about 4900 mPa·s, or to about 4700 mPa·s, or to about 4500 mPa·s, or to about 4300 mPa·s, or to about 4100 mPa·s, or to about 3900 mPa·s, or to about 3700 mPa·s, or to about 3500 mPa·s, or to about 3300 mPa·s, or to about 3100 mPa·s, or to about 2900 mPa·s, or to about 2500 mPa·s, or to about 2300 mPa·s measured at 25°C. In an embodiment, the viscosity is from about 3000 mPa·s to about 4500 mPa·s measured at 25°C.

### Conditioning pre-treatment

Aspects of the present invention employ or relate to a conditioning pre-treatment. In an embodiment, the conditioning pre-treatment is a conditioning formulation and comprises a conditioning agent. In an embodiment, the conditioning agent selected from the group consisting of: silicone compounds; cationic surfactants; and mixtures thereof. Such conditioning agents are described herein.

### Neutralising formulation

Aspects of the present invention employ or relate to a neutralising formulation. In an embodiment, the neutralising formulation has a pH of from about pH 2.0 to about pH 7.0, or from about pH 2.5 to about pH 6.5, or from about pH 3.0 to about pH 6.0, or from about pH 3.5 to about pH 5.5. In an embodiment, the neutralising formulation comprises an oxidising agent. In an embodiment, the oxidising agent is selected from the group consisting of: peroxides; persulfates; and mixtures thereof. In an embodiment, the neutralising formulation comprises from from about 1% to about 10%, or about 2% to about 5% oxidising agent.

### Finishing formulation

Aspects of the present invention employ or relate to a finishing formulation. In an embodiment, the finishing formulation is a conditioning formulation and comprises a conditioning agent. In an embodiment, the conditioning agent selected from the group consisting of: silicone compounds; cationic surfactants; and mixtures thereof. Such conditioning agents are described herein.

### 2^{nd} aspect

The features of the second aspect are stated in the SUMMARY OF THE INVENTION above. The second aspect relates to a hair straightening method. The hair straightening method comprises analysing hair health and/or hair type. In an embodiment, analysing hair health and/or hair type comprises: assessing hair dryness, assessing hair volume, assessing hair curliness, assessing hair colour, assessing hair fibre thickness and brittleness, checking for split ends, and combinations thereof. In an embodiment, analysing hair health and/or hair type is carried out with the naked eye. In an embodiment, the hair straightening method comprises applying a conditioning pre-treatment to the hair. In an embodiment, the conditioning pre-treatment is a conditioning formulation. The conditioning pre-treatment may be employed where the analysing hair health and/or hair type results in: above normal hair dryness, below normal hair fibre thickness and above normal hair fibre brittleness, split ends, and combinations thereof. The hair straightening method comprises mixing together a conditioning formulation and a straightening formulation to form a hair conditioning composition. The conditioning formulation may be as described herein. The straightening formulation may be as described herein. In an embodiment, straightening formulation comprises a reducing agent. Reducing agents and their amounts are described herein. The hair conditioning composition may be as described herein. The hair straightening method comprises allowing the hair conditioning composition to remain on the hair for a development time x. In an embodiment, development time x is from about 1 min to about 45 min, or from about 5 min to about 35 min, or from about 10 min to about 30 min. The hair straightening method comprises drying the hair. In an embodiment, the drying the hair comprises employing a blow dryer. In an embodiment, the drying the hair comprises employing a blow dryer for at least 10 minutes. The hair straightening method comprises mechanically straightening the hair using a temperature of from about 80°C to about 250°C. In an embodiment, the temperature is from about 90°C to about 240°C, or from about 100°C to about 230°C, or from about 110°C to about 220°C, or from about 120°C to about 210°C, or from about 130°C to about 200°C, or from about 140°C to about 190°C. Lower temperatures, particularly below 220°C are preferred in view of reduced hair damage. The hair straightening method comprises applying a neutralising formulation to the hair. A neutralising formulation is described herein. In an embodiment, the hair straightening method comprises applying a finishing formulation to the hair. A finishing formulation is described herein. In an embodiment, the finishing formulation is a conditioning formulation and comprises a conditioning agent. Conditioning agents and their amounts are described herein.

### 3^{rd} aspect

The features of the third aspect are stated in the SUMMARY OF THE INVENTION above. The third aspect relates to a process for obtaining a hair conditioning composition. The process comprises analysing hair health and/or hair type. Such analysing is described in the second aspect. The process comprises selecting and providing a conditioning formulation and a straightening formulation. Such conditioning formulations and straightening formulations are described herein. The process comprises mixing together the conditioning formulation and the straightening formulation in proportions tailored to the hair health and/or hair type. In an embodiment, the proportions tailored to the hair health and/or hair type are ratios of conditioning formulation to straightening formulation selected from 4:1 to 1:4. In an embodiment, the proportions tailored to the hair health and/or hair type are ratios of conditioning formulation to straightening formulation selected from 1:1; 2:1; 3:1; 1:3 and 1:3. In an embodiment, ratio of conditioning formulation to straightening formulation being from 1:1 to 4:1, or 2:1 to 4:1 is employed where the analysing hair health and/or hair type results in: above normal hair dryness, below normal hair fibre thickness and above normal hair fibre brittleness, split ends, , non-natural or non-environmentally-induced hair colour, and combinations thereof. In an embodiment, ratio of conditioning formulation to straightening formulation being from 1:4 to 1:1, or 1:4 to 1:2 is employed where the analysing hair health and/or hair type results in: below normal hair dryness, above normal hair curliness, and combinations thereof. "Average" as used in this paragraph means the average condition of hair from at least 100 individuals. In an embodiment, the mixing together the conditioning formulation and the straightening formulation results in the hair conditioning composition of the first aspect.

### 4^{th} aspect

The features of the fourth aspect are stated in the SUMMARY OF THE INVENTION above. The fourth aspect relates to a composition directly obtained by the process of the third aspect. The composition may be the same as the hair conditioning composition of the first aspect or otherwise described herein.

### 5^{th} aspect

The features of the fifth aspect are stated in the SUMMARY OF THE INVENTION above. The fifth aspect relates to a hair straightening kit. In an embodiment, the kit is a hair straightening kit comprising: (a) a conditioning pre-treatment; (b) a straightening formulation comprising a reducing agent; (c) a conditioning formulation comprising conditioning agent selected from the group consisting of: silicone compounds; cationic surfactants; and mixtures thereof; (d) a conditioning infusion and/or a conditioning oil; (e) a neutralising formulation; (f) optionally a finishing formulation. The descriptions of the formulations in the above sections apply to the fifth aspect *mutatis mutandis.* In an embodiment, kit comprises a conditioning oil. In an embodiment, the conditioning oil comprises a silicone polymer and an aliphatic hydrocarbon having from 8 to 18, or from 10 to 14 carbon atoms.

### 6^{th} aspect

The sixth aspect of the invention relates to various uses of the composition of the first aspect or the fourth aspect. An embodiment relates to the use of the composition, according to the first aspect or the fourth aspect, for improved hair shine. An embodiment relates to the use of the hair conditioning composition, according to the first aspect or the fourth aspect, on already dyed hair. The phrase "already dyed hair" as used herein means hair that has already been subjected to a hair dyeing, hair bleaching or hair colouring treatment, particularly via the employment of dye chemistry, peroxides or persulfates, particularly oxidative dyes. An embodiment relates to the use of the hair conditioning composition, according to the first aspect, for depositing a terminal aminosilicone onto hair, or providing improved deposition of a terminal aminosilicone onto hair. The terminal aminosilicone may be as described herein. An embodiment relates to the use of the composition, according to the first aspect or the fourth aspect, for improved hair combing, preferably for reduced combing force. An embodiment relates to the use of the composition, according to the first aspect or the fourth aspect, for application onto already straightened hair.

### 7^{th} aspect

The seventh aspect of the invention relates to a method of treating already dyed hair comprising applying a hair straightening composition to the already dyed hair, wherein the hair straightening composition comprises:
- from about 2.0% to about 9.0% reducing agent;
- from about 0.5% to about 1.5% alkalising agent;
- from about 2.0% to about 10% conditioning agent;
- a buffering agent;
wherein the composition has a pH of from about pH 7.5 to about pH 9.5;
wherein the composition has a viscosity of about 500 mPa·s to about 5500 mPa·s measured at 25°C. The phrase "already dyed hair" as used herein means hair that has already been subjected to a hair dyeing, hair bleaching or hair colouring treatment, particularly via the employment of dye chemistry, peroxides or persulfates, particularly oxidative dyes. In an embodiment, the reducing agent is selected from the group consisting of ammonium thioglycolate, ammonium thiolactate, and mixtures thereof. In an embodiment, the composition is substantially free of an additional reducing agent capable of reducing disulfide bonds in keratin fibres. In an embodiment, the hair straightening composition is according to the first aspect or otherwise described herein.

### Other aspects

Another aspect of the invention relates to a method of treating already straightened hair comprising applying a hair straightening composition to the already straightened hair, wherein the hair straightening composition comprises:
- from about 2.0% to about 9.0% reducing agent, wherein the reducing agent is selected from the group consisting of ammonium thioglycolate, ammonium thiolactate and mixtures thereof;
- from about 0.5% to about 1.5% alkalising agent;
- from about 2.0% to about 10% conditioning agent;
- a buffering agent;
wherein the composition has a pH of from about pH 7.5 to about pH 9.5;
wherein the composition has a viscosity of about 500 mPa·s to about 5500 mPa·s measured at 25°C;
wherein the composition is substantially free of an additional reducing agent capable of reducing disulfide bonds in keratin fibres. The phrase "already straightened hair" as used herein means hair that has already been subjected to a straightening treatment, particularly by the employment of reducing agents or crosslinking chemistry and/or via the application of straightening irons, particularly heated straightening irons. In an embodiment, the hair conditioning composition is as per the first aspect or otherwise described herein.

### EXAMPLES

The following examples further describe and demonstrate embodiments within the scope of the present invention. The examples are given solely for the purpose of illustration, and are not to be construed as limitations of the present invention since many variations thereof are possible without departing from its scope.

Straightening formulation examples:

| Ingredient / Example | 1 | 2 | 3 | 4 |
|---|---|---|---|---|
| Ammonium thioglycolate (48% in water) | 20 | - | 19 | - |
| Diammonium dithioglycolate (59% in water) | 10 | - | 8 | - |
| Ammonium thioglycolate (50% in water) | - | 21 | - | 20 |
| Diammonium dithiolactate (50% in water) | - | 9 | - | 10 |
| Ammonia (28%) | 1.9 | 1.8 | 1.9 | 1.8 |
| Polyquaternium-6 (40% in water) | - | 0.5 | 1.0 | 1.1 |
| Polyquaternium-7 (40 % in water) | 1.0 | 0.5 | - | - |
| Ceteareth-25 | - | 2.5 | 2.5 | - |
| Cetearyl alcohol | - | 5 | 6 | 4 |
| Cetyl alcohol | 5 | - | - | 2 |
| Ammonium bicarbonate | 1.3 | 1.2 | 1.3 | 1.1 |
| Beeswax | - | - | 1.0 | - |
| Preservatives | 0.1 | 0.2 | 0.15 | 0.25 |
| Perfume | 0.2 | 0.1 | 0.07 | 0.1 |
| Deionised water | QSP 100 | QSP 100 | QSP 100 | QSP 100 |

Conditioning formulation examples:

| Ingredient / Example | A | B | C | D |
|---|---|---|---|---|
| Terminal aminosilicone* | 3.0 | 3.5 | 2.8 | 4.0 |
| Behentrimonium chloride | - | 2.9 | 3.0 | 0.1 |
| Cetrimonium chloride | 2.0 | - | - | 3.0 |
| Cetyl alcohol | 2.0 | 1.5 | - | 1.9 |
| Stearyl alcohol | 4.0 | 5.0 | 6.0 | 4.5 |
| D/L-Panthenol | 1 | 0.8 | - | 1.3 |
| Creatine | 0.2 | - | - | 0.25 |
| Preservatives | 0.5 | 0.4 | 0.5 | 0.4 |
| Perfume | 0.5 | 0.6 | 0.4 | 0.2 |
| Deionised water | QSP 100 | QSP 100 | QSP 100 | QSP 100 |

| | | | | |
|---|---|---|---|---|
| KEY: * = selected from the group consisting of bis-aminomethyl dimethicone, bis-aminoethyl dimethicone, bis-aminopropyl dimethicone, bis-aminobutyl dimethicone, and mixtures thereof. | | | | |

Hair conditioning composition examples:

| Formulation (see above) / Example | i | ii | iii | iv | v | vi |
|---|---|---|---|---|---|---|
| Straightening formulation 1 | 70 | - | 50 | - | 30 | - |
| Straightening formulation 2 | - | 59 | - | - | - | - |
| Straightening formulation 3 | - | - | - | - | - | 20 |
| Straightening formulation 4 | - | - | - | 50 | - | - |
| Conditioning formulation A | 30 | - | - | - | - | 80 |
| Conditioning formulation B | - | 39 | - | - | - | - |
| Conditioning formulation C | - | - | - | 49 | 70 | - |
| Conditioning formulation D | - | - | 50 | - | - | - |
| Conditioning oil (comprises isododecane and polydimethylsiloxane) | - | 2 | - | 1 | - | - |
| Total | 100% | 100% | 100% | 100% | 100% | 100% |
| Used when... | a | b | c | d | e | f |

| | | | | | | |
|---|---|---|---|---|---|---|
| KEY: a = when hair fibres are above average thickness or where the hair has excellent condition or where the hair has not been dyed; b = normal/average hair; c and d = where the hair has been dyed and/or where less than 30% of the hair fibres on the head of hair have been highlighted; e = where the hair fibres are finer than average or where the hair is drier or more damaged than average or where the hair has been coloured or where less than 50% of the hair fibres on the head of hair have been highlighted; f = where the hair is especially drier than average or where the hair is especially damaged with many split ends or where 60% or more of the hair fibres on the head of hair have been highlighted. | | | | | | |

In cases a to f, a conditioning pre-treatment, for example the conditioning oil as featured in the table, may be applied. This may be useful where the head of hair in general is in good condition, but the ends are dry and may have splitting.

### Technical Data

Figs 1 and 2 shows data for the force when combing hair for various samples. Fig 1 is wet combing and Fig 2 is dry combing. Such combing experiments are performed with 2x pre-bleached Caucasian hair tresses and a robotic combing device. The bleaching simulates typical hair damage of hair on head. The combing force is measured in Newtons (N) - see axis y - and represents friction of hair against comb, hair against hair as well as disentangle force, which is necessary to separate tangled hair. Lower combing force is proportional to higher conditioning and reduced hair damage during combing. Both wet combing and dry combing are important for consumers and stylists since after washing, the hair is usually combed whilst it is still wet, and then further combed after drying. For each tress, 20 combs are perfomed and the results are the mean readings per comb per tress (6 tresses are used per sample). The error bars represent the standard deviation calculated using the T-test.

Fig 3 shows data for the shine percentage on various samples. In this experiment, the hair tress is placed on a cylinder, which is illuminated under definite conditions. The angular distribution of the reflected light is measured and the ratios of specularly and diffusely reflected light are determined. The higher the percentage of directly reflected light and the lower the percentage of diffusely scattered light, the more intense the shine. The shine band measured. Generally a narrow angular distribution (full width at half maximum) indicates more shine while a wide angular distribution means less shine. A higher shine percentage is proportional to increased hair shine.

In Figs 1-3, 2g hair tresses are used and the following are compared: control sample tresses (no treatment), tresses treated with a straightening formulation only (sample 2), tresses treated with a hair conditioning composition according to the present invention (sample 1). The straightening formulation used for sample 2 comprises: cetearyl alcohol 6%; ceteareth-25 2.5%; isopropyl myristate 2%; polyquaternium-6 0.4%; ammonia 5.25%; ammonium hydroxide 0.5%; ammonium bicarbonate 1.3%; diammonium dithioglycolate 4%; ammonium thioglycolate 11%, water. The hair conditioning composition according to the present invention used for sample 1 is created by mixing the straightening formulation used for sample 2 with a conditioning formulation comprising: cetyl alcohol 1.9%; stearyl alcohol 4.6%; behentrimonium chloride 2.8%; 10000 cPs terminal aminosilicone 2.5%; fragrance 0.5%; panthenol DL 1%; water. The treated tresses are subsequently treated with a neutraling formulation where the neutralising formulation comprises from 2% to 5% hydrogen peroxide as oxidising agent.

Conclusions from the data: the present invention provides improved wet and dry combing and also increase shine versus controls.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair conditioning composition comprising:
- from about 2.0% to about 9.0% reducing agent, wherein the reducing agent is selected from the group consisting of: ammonium thioglycolate; ammonium thiolactate; and mixtures thereof;
- from about 0.5% to about 1.5% alkalising agent;
- from about 2.0% to about 10% conditioning agent selected from the group consisting of: silicone compounds; cationic surfactants; and mixtures thereof;
- a buffering agent;
wherein the composition has a pH of from about pH 7.5 to about pH 9.5;
wherein the composition has a viscosity of from about 500 mPa·s to about 5500 mPa·s measured at 25°C;
wherein the composition is substantially free of an additional reducing agent capable of reducing disulfide bonds in keratin fibres.

2. The composition according to claim 1, wherein the composition comprises a mixture of polydimethylsiloxane and terminal aminosilicone.

3. The composition according to any of the preceding claims, wherein the cationic surfactant is according to Formula II 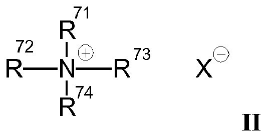 wherein at least one of R⁷¹, R⁷², R⁷³ and R⁷⁴ is an aliphatic group having from 16 to 24 carbon atoms; wherein the remainder of R⁷¹, R⁷², R⁷³ and R⁷⁴ are independently selected from the group consisting of aliphatic groups having from 1 to 4 carbon atoms; wherein X is selected from the group consisting of: chloride or sulfate.

4. The composition according to any of the preceding claims, wherein said additional reducing agent is selected from the group consisting of: cysteine, monoethanolamine thioglycolate, thiosulfates, sulfites, bisulfites, carbonyl group-containing compounds and mixtures thereof.

5. The composition according to any of the preceding claims, wherein the composition is substantially free of monoethanolamine.

6. The composition according to any of the preceding claims, wherein the composition comprises a thickening polymer, and wherein the thickening polymer is a polyquaternium compound.

7. The composition according to any of the preceding claims, the viscosity is from about 3000 mPa·s to about 4500 mPa·s measured at 25°C.

8. A hair straightening method comprising:
i. analysing hair health and/or hair type;
ii. optionally applying a conditioning pre-treatment to the hair;
iii. mixing together a conditioning formulation and a straightening formulation to form a hair conditioning composition;
iv. applying the hair conditioning composition to hair;
v. allowing the hair conditioning composition to remain on the hair for a development time x;
vi. rinsing and drying the hair;
vii. mechanically straightening the hair using a temperature of from about 80°C to about 250°C;
viii. applying a neutralising formulation to the hair;
ix. rinsing the hair;
x. optionally applying a finishing formulation to the hair.

9. A process for obtaining a hair conditioning composition, comprising:
(I) analysing hair health and/or hair type;
(II) selecting and providing a conditioning formulation and a straightening formulation;
(III) mixing together the conditioning formulation and the straightening formulation in proportions tailored to the hair health and/or hair type.

10. The composition directly obtained by the process of claim 9.

11. A hair straightening kit comprising
(a) optionally a conditioning pre-treatment;
(b) a straightening formulation comprising a reducing agent;
(c) a conditioning formulation;
(d) optionally a conditioning infusion and/or a conditioning oil;
(e) optionally a neutralising formulation;
(f) optionally a finishing formulation.

12. Use of the composition, according to any of claims 1-7 and 10, for improved hair shine.

13. Use of the composition, according to any of claims 1-7 and 10, for improved hair combing, preferably for reduced combing force.

14. Use of the composition, according to any of claims 1-7 and 10, for application onto already straightened hair.

15. A method of treating already dyed hair comprising applying a hair straightening composition to the already dyed hair, wherein the hair straightening composition comprises:
- from about 2.0% to about 9.0% reducing agent;
- from about 0.5% to about 1.5% alkalising agent;
- from about 2.0% to about 10% conditioning agent;
- a buffering agent;
wherein the composition has a pH of from about pH 7.5 to about pH 9.5;
wherein the composition has a viscosity of about 500 mPa·s to about 5500 mPa·s measured at 25°C.
